Europäisches Patentamt

European Patent Office

Office européen des brevets

(19)

(11) Numéro de publication: **0 168 897**
A2

# (12) DEMANDE DE BREVET EUROPEEN

(21) Numéro de dépôt: 85201164.2

(22) Date de dépôt: 11.07.85

(51) Int. Cl.⁴: **A 23 F 5/02,** A 61 K 7/00

(30) Priorité: 18.07.84 BE 213347
11.02.85 BE 214496

(43) Date de publication de la demande: 22.01.86
Bulletin 86/4

(84) Etats contractants désignés: **AT BE CH DE FR GB IT LI LU NL SE**

(71) Demandeur: **Storme, Francis, rue du Beau Site 74, B-7700 Mouscron (BE)**

(72) Inventeur: **Couckhuyt, Noel, Emiel Neirynckstraat 7, B-8760 Lendelede (BE)**

(74) Mandataire: **Pieraerts, Jacques et al, Bureau Gevers S.A. rue de Livourne 7, Bte. 1, B-1050 Bruxelles (BE)**

(54) **Produit solide à base de grains de cafés, boisson, composition alimentaire ou à usage externe à base dudit solide et procédé d'obtention.**

(57) L'invention concerne un produit solide provenant de la mouture de grains de café vert, destiné à être utilisé en vue de la constitution de boissons et de produits alimentaires solides, ainsi qu'à une utilisation en usage externe. L'invention concerne également le procédé de réalisation de ce produit.

EP 0 168 897 A2

ACTORUM AG

Cette invention concerne un produit solide présentant une granulométrie située entre celle de la farine et celle de la semoule et obtenu à partir de grains de café.

L'invention concerne également un procédé d'obtention d'un produit à base de grains de café.

Le but de l'invention est de procurer un produit de conception entièrement nouvelle qui, dans ses applications à usage interne et externe, présente des propriétés tout à fait remarquables.

Conformément à l'invention le produit solide précité provient de la mouture de grains de café vert, destinée à être utilisée en vue de la constitution de boissons et de produits alimentaires solides ainsi qu'à une utilisation en usage externe.

Selon une forme de réalisation appliquée de préférence on part de grains de café vert, on soumet ces grains à un processus de séchage, et immédiatement après à un refroidissement, et on mout ces grains entre deux meules.

Une particularité remarquable de l'invention réside dans le fait qu'on effectue la mouture entre deux meules disposées verticalement.

D'autres détails et avantages de l'invention ressortiront de la description qui sera donnée ci-après du produit solide, des boissons et compositions à base de ce produit, ainsi que du procédé de réalisation de celui-ci, selon l'invention. Cette description n'est donnée qu'à titre d'exemple et ne limite pas l'invention. Un but tout particulier de l'invention est de réaliser un produit qui est à la base de compositions solides, de boissons ou de produits cosmétiques à usage externe dont le principe actif est la caféïne.

Le demandeur a en effet constaté les effets tout à fait exceptionnels de la caféïne lorsque celle-ci n'a pas été modifié dans sa structure ni "poluée" par des hydrocarbures suite à une torréfaction.

En effet, des tests chimiques effectués par le demandeur n'ont pas porté sur les seuls effets de la caféine mais sur le complexe formé par la caféine et les hydrocarbures lorsque les grains de café ont été préalablement soumis à une torréfaction.

La caféine dont la dénomination scientifique est 1-3-7-trimethylxanthine joue un rôle important dans la formation de la choline en partant de l'étanolamine. La choline est en effet une importante substance lipotrope qui empêche la formation et l'accumulation anormale de graisses dans les tissus. C'est en cédant le groupe $CH_3$ à l'étanolamine que se forme la choline.

Le café torrifié modifie la caféine qui se trouve attaquée par les hydrocarbures qui adhèrent à l'étanolamine qui ne forme ainsi pas la choline mais un produit toxique différent agissant de manière défavorable sur l'organisme. En réduisant à l'état de farine, des grains de café vert, la caféine pure et les oligo-éléments qui se retrouvent également dans les grains de café agissent sur l'organisme d'une façon totalement inconnue. La caféine pure, non chargée d'hydrocarbures, a un effet différent sur les centres nerveux, les muscles lisses, de telle sorte qu'on peut estimer que cette caféine à l'état pur a un effet spasmolytique sur les intestins et bénéfique dans les cas de crises, de migraine.

Il a donc été constaté les effets surprenants de la caféine à l'état pur. Les grains de café vert moulus engendrent un produit dans lequel la structure de la caféine n'est pas modifiée.

En dehors de la caféine on retrouve encore les nombreux oligo-éléments auxquels il a déjà été fait allusion, c.à.d. les oligo-éléments dont les traces ont été retrouvées dans les proportions indiquées ci-après:

Manganèse                     32,7 microgramme/g

| Fer | 134,7 microgramme/g |
| --- | --- |
| Zinc | 10,6 microgramme/g |
| Strontium | 6,7 microgramme/g |
| Rubidium | 34,6 microgramme/g |
| Cuivre | 16,6 microgramme/g |
| Brome | 0,5 microgramme/g |
| Plomb | 0,9 microgramme/g |

En dehors de ces oligo-éléments on retrouve encore des vitamines du groupe B ci-après et dans les proportions ci-dessous:

| Vitamine B1 (Thiamine) CHNOS | 0,21 | milligramme |
| --- | --- | --- |
| Vitamine B2 (Riboflavine) CHNC | 0,23 | milligramme |
| Acide pantothénique CHON | 1,0 | milligramme |
| Choline | 59,0 | milligramme |
| Acide folique | 0,020 | milligramme |
| Facteur Citrovorum | 0,012 | milligramme |
| Vitamine B6 | 0,143 | milligramme |
| Vitamine B12 | 0,00011 | milligramme |

Les quantités indiquées ci-dessus ont été retrouvées dans une quantité de 100 grammes de café vert.

Il a été constaté de manière expérimentale que les oligo-éléments et les vitamines citées ci-dessus favorisent l'anti-toxicité du produit, sont diurétiques et diminuent la teneur en sucre dans le sang.

Toutes les espèces de café vert sont utilisables dans le cadre du procédé selon l'invention en vue de réaliser un produit solide pouvant constituer la base de boissons, de compositions alimentaires ou être introduit dans des cosmétiques destinés à l'usage externe.

Les phases principales du procédé sont les suivantes:

Phase 1 Sélection de grains de café vert préalablement lavés; cette sélection peut être éventuellement exécutée en faisant usage d'un appareil équipé d'un oeil électronique.

Phase 2 Procédé de séchage à une température qui est située idéalement entre ± 55 et 65°C.

Phase 3 Refroidissement qui intervient immédiatement après le processus de séchage, notamment en vue d'empêcher que les grains de café ne reprennent de l'humidité.

Phase 4 Nettoyage des grains de café encore entières et séchées.

Phase 5 Seconde nettoyage visant principalement l'élimination de petites pierres et d'autres impuretés.

Phase 6 Mouture qui a lieu entre des meules disposées verticalement.

Phase 7 Refroidissement et évacuation vers un espace de stockage tel qu'un silo.

Phase 8 Les grains de café vert moulus sont soumis à un processus de tamisage qui y fait apparaître au moins trois produits de structure différente et cela dans les trois phases ci-après:

Phase 9 La mouture qui apparaît en premier lieu a une granulométrie qui permet un bon travail du produit obtenu.

Phase 10 qui constitue en un blutage supplémentaire, produit un mélange dans lequel apparaît la farine et un produit présentant un grain plus prononcé.

Phase 11 Second blutage qui ne fait plus apparaître qu'une farine.

Après les huit phases citées ci-dessus on obtient de manière strictement naturelle un produit qui n'a subiaucun traitement chimique. Le produit obtenu présente une odeur particulièrement reconnaissable.

Les produits ayant les diverses granulométries qui apparaissent lors des phases 9, 10 et 11 peuvent être missur le marché sous diverses formes.

Les produits moulus présentant les propriétés citées ci-dessus peuvent servir de base de la fabrication de biscuits, être additionnés à d'autres matières de base. Les fèves moulues peuvent également être à la base de boissons de tous types et notamment de boissons rafraîchissantes. Elles peuvent également être à la base des produits les plus divers aussi bien destinés à l'usage externe qu'à l'usage interne et cela chez l'homme et chez l'animal. Elles peuvent être à la base de produits cosmétiques en faisant usage d'un excipient approprié.

## REVENDICATIONS

1. Produit solide présentant une granulométrie située entre celle de la farine et celle de la semoule et obtenu à partir de grains de café, caractérisé en ce qu'il provient essentiellement de la mouture de grains de café vert, destiné à être utilisé en vue de la constitution de boissons et de produits alimentaires solides, ainsi qu'à une utilisation en usage externe.

2. Boisson réalisée en faisant usage d'un produit conforme à la revendication 1.

3. Produits solides tels que biscuits et analogues consistant essentiellement, ou en partie, en un produit solide conforme à la revendication 1.

4. Composition à base d'un produit conforme à la revendication 1 et destiné à l'usage interne ou externe, entre autres, cosmétique.

5. Procédé de réalisation d'un produit à base de grains de café, caractérisé en ce qu'on part de grains de café vert, on soumet ces grains à un processus de séchage et, immédiatement après, à un refroidissement et une mouture entre deux meules.

6. Procédé selon la revendication 5, caractérisé en ce que le séchage des grains de café vert est exécuté à une température située entre 55 et 65°C.

7. Procédé selon l'une quelconque des revendications 5 et 6, caractérisé en ce qu'on effectue la mouture entre deux meules disposées verticalement.

8. Procédé selon l'une quelconque des revendications 5 à 7, caractérisé en ce que le produit résultant de la mouture est soumis à plusieurs opérations de blutage jusqu'à obtention d'un produit ayant une consistance allant de celle de la farine à celle de la semoule.